# EUROPEAN PATENT SPECIFICATION

(11) **EP 3 991 632 B1**
(45) Date of publication and mention of the grant of the patent: **11.12.2024**
(21) Application number: 21204665.0
(22) Date of filing: 26.10.2021
(51) Int. Cl.: A61B 1/00, A61B 1/005, A61B 1/06

(54) **ENDOSCOPE SYSTEM AND ENDOSCOPE DEVICE**
ENDOSKOPSYSTEM UND ENDOSKOPVORRICHTUNG
SYSTÈME D'ENDOSCOPE ET DISPOSITIF D'ENDOSCOPE

(30) Priority: 28.10.2020 JP 2020180681
(43) Date of publication of application: 04.05.2022
(73) Proprietor: FUJIFILM Corporation, Tokyo 106-8620 (JP)
(72) Inventor: KIMURA, Yuya, Kanagawa (JP)
(74) Representative: Dehns Germany Partnerschaft mbB

(56) References cited:
- EP-A1- 3 603 483
- WO-A1-2009/069395
- WO-A1-2020/039800
- JP-A- 2014 230 612
- JP-B2- 6 626 837
- US-A1- 2008 009 714

## Description

### BACKGROUND OF THE INVENTION

### 1. Field of the Invention

The present invention relates to an endoscope system and an endoscope device in which a part to be observed can be easily grasped in endoscopy targeting a part having poor visibility.

### 2. Description of the Related Art

In recent years, in endoscopy in the medical field, endoscope systems have been widespread, which can efficiently perform the endoscopy by grasping the shape of an insertion part in a body cavity of a subject and utilizing information on the shape of the insertion part.

For example, in JP2014-230612A, a virtual movement line of an endoscope insertion part in a body cavity is calculated on the basis of a CT image obtained by a computed tomography (CT) apparatus, and in a case where the endoscopy is actually performed on a lesion estimated from the CT image, the operation up to the position of the lesion is supported by utilizing the virtual movement line created on the basis of the CT image.

Additionally, in WO2009/069395A, an endoscope of WO2009/069395A detects the position and orientation of a distal end of an insertion part in an endoscope having a plurality of coils built in the insertion part placed in a magnetic field to store the position and the orientation as instructional information, and supports operations on the basis of the stored instructional information. Document EP3603483 A1 describes an endoscope system comprising: a processor that acquires shape data of an insertion part of an endoscope in a body cavity in an examination using the endoscope with movement of a distal end of the insertion part; and a memory that stores the shape data, wherein the processor stores the shape data at a position of an object to be observed in a first examination as first shape data in the memory, and determines that the distal end of the insertion part has reached the position of the object to be observed and provides notification of a result of determination in a case where the shape data acquired in a second examination after the first examination matches the first shape data. The documents US2008/009714 A1 and WO 2020/039800 A1 describe similar endoscopic systems.

### SUMMARY OF THE INVENTION

In endoscopy of the large intestine, in a case where a part to be observed that has been observed once is observed again, in the method of searching for a target part to be observed by relying on an observation image of the part to be observed and comments on the position of the part to be observed, it may be difficult to grasp the position of the part to be observed due to the lack of landmarks in the lumen or a decrease in visibility caused by residue. For that reason, there has been an eager desire for a method of making it easier to grasp the position of the target part to be observed by utilizing the acquired examination information even in the endoscopy targeting a part having poor visibility.

An object of the present invention is to provide an endoscope system and an endoscope device in which a part to be observed can be easily grasped in endoscopy targeting a part having poor visibility.

The present invention has been made to solve the above problems, and the endoscope system of the present invention comprises a processor that acquires shape data of an insertion part of an endoscope in a body cavity in an examination using the endoscope with movement of a distal end of the insertion part; and a memory that stores shape data, and the processor stores the shape data at a position of an object to be observed in a first examination as first shape data in the memory, and determines that the distal end of the insertion part has reached the position of the object to be observed and provides notification of a result of examination in a case where the shape data acquired in a second examination after the first examination matches the first shape data.

It is preferable that the endoscope system further comprises a display, and the processor stores an observation image obtained by imaging the object to be observed and the first shape data in association with each other in the memory, and displays the observation image on the display in a case where the shape data acquired in the second examination matches the first shape data.

It is preferable that the processor compares the shape data with the first shape data each time the shape data is acquired, in the second examination.

It is preferable that the processor stores the shape data, which has matched the first shape data, in the second examination as second shape data in the memory.

The processor stores in the memory an arrival time, which is a time at which the distal end of the insertion part reaches the position of the object to be observed after the insertion part is inserted into the body cavity, and performs a notification on the basis of the arrival time.

It is preferable that the processor displays diagnosis information of the object to be observed, which is associated with the first shape data, on the display.

It is preferable that the endoscope system further comprises a light source control processor, and the light source control processor controls a light source that emits first illumination light and second illumination light having a different emission spectrum from the first illumination light.

It is preferable that the first illumination light is white light, and the observation image is an image captured by irradiating the object to be observed with the first illumination light.

It is preferable that the light source control processor causes the first illumination light to be emitted in a first illumination period, causes the second illumination light to be emitted in a second illumination period, and in a case where the first illumination period and the second illumination period are automatically and alternately switched and in a case where a predetermined illumination period is defined as one frame, controls the first illumination light in a first light emission pattern having a first A light emission pattern in which a total number of frames obtained by summing the frames in each first illumination period is the same in all the first illumination periods, and a first B light emission pattern in which a total number of frames in one of the first illumination periods is different from the total number of frames in at least one other first illumination period.

It is preferable that the light source control processor controls the second illumination light in a second light emission pattern, the second light emission pattern has a second A light emission pattern, a second B light emission pattern, a second C light emission pattern, and a second D light emission pattern, the second A light emission pattern is a light emission pattern in which the total number of frames in each second illumination period is the same in all the second illumination periods and an emission spectrum of the second illumination light in each second illumination period is the same in all the second illumination periods, the second B light emission pattern is a light emission pattern in which the total number of frames in each second illumination period is the same in all the second illumination periods, and the emission spectrum of the second illumination light in one of the second illumination periods is different from the emission spectrum of the second illumination light in at least one other second illumination period, the second C light emission pattern is a light emission pattern in which a total number of frames in one of the second illumination periods is different from the total number of frames in at least one other second illumination period and the emission spectrum of the second illumination light in each second illumination period is the same in all the second illumination periods, and the second D light emission pattern is a light emission pattern in which the total number of frames in one of the second illumination periods is different from the total number of frames in at least one other second illumination period and the emission spectrum of the second illumination light in one of the second illumination periods is different from the emission spectrum of the second illumination light in at least one other second illumination period.

An endoscope device of the present invention comprises a processor that acquires shape data of an insertion part of an endoscope in a body cavity in an examination using the endoscope with movement of the insertion part of the endoscope; and a memory that stores shape data, and the processor stores the shape data at a position of an object to be observed in a first examination as first shape data in the memory, and determines that the distal end of the insertion part has reached the position of the object to be observed and provides notification a result of determination in a case where the shape data acquired in a second examination after the first examination matches the first shape data.

According to the present invention, in endoscopy targeting a part having poor visibility, attention is called at an appropriate position with respect to a target part to be observed. Thus, the endoscope system and the endoscope device that make it easy to grasp the part to be observed can be provided.

### BRIEF DESCRIPTION OF THE DRAWINGS

Fig. 1 is a block diagram schematically showing an overall configuration of an endoscope system of the present invention.
Fig. 2 is an explanatory diagram showing a schematic configuration of an endoscope of the present invention.
Fig. 3 is an explanatory diagram illustrating a first A light emission pattern in a first light emission pattern and a second A light emission pattern in a second light emission pattern.
Fig. 4 is an explanatory diagram illustrating a first B light emission pattern in the first light emission pattern.
Fig. 5 is an explanatory diagram illustrating a second B light emission pattern in the second light emission pattern.
Fig. 6 is an explanatory diagram illustrating a second C light emission pattern in the second light emission pattern.
Fig. 7 is an explanatory diagram illustrating a second D light emission pattern in the second light emission pattern.
Fig. 8 is a block diagram showing respective functions of a processor in a processor device of the first embodiment.
Fig. 9 is an explanatory diagram showing stored information stored in a shape data storage unit according to the first embodiment.
Fig. 10 is a first explanatory diagram illustrating the functions of a shape data collation unit according to the first embodiment.
Fig. 11 is an explanatory diagram showing an example of notification in the first embodiment.
Fig. 12 is an explanatory diagram showing an example of displaying a previously observed image in the first embodiment.
Fig. 13 is a second explanatory diagram illustrating the functions of the shape data collation unit according to the first embodiment.
Fig. 14 is an explanatory diagram showing stored information stored in a shape data storage unit according to a second embodiment.
Fig. 15 is an explanatory diagram illustrating the functions of a shape data collation unit according to the second embodiment.
Fig. 16 is an explanatory diagram illustrating the functions of a shape data collation unit according to a third embodiment.

### DESCRIPTION OF THE PREFERRED EMBODIMENTS

### First Embodiment

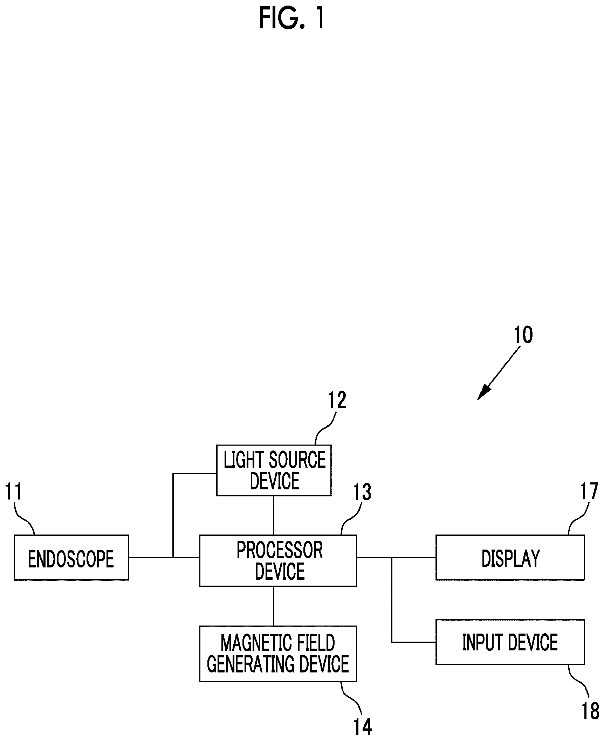

The endoscope system 10 of the present invention is a system that makes it easy to find a part to be observed that has been observed in the past, in the follow-up observation of observing the part to be observed including an object to be observed using an endoscope. As the object to be observed, in addition to lesions and tumors, inflamed spots and difficult-to-insert parts are main objects. As shown in Fig. 1, the endoscope system 10 includes an endoscope 11, a light source device 12, a processor device 13, a magnetic field generating device 14, and a display 17 and an input device 18 connected to the processor device 13.

Since a computer constituting the processor device 13 has a general hardware configuration, the illustration of each hardware is omitted, but the computer has a central processing unit (CPU), a memory, a storage device, and an input and output interface (I/F). The display 17 and the input device 18 are connected to the input and output I/F. In addition, the processor device 13 corresponds to an endoscope device of the present invention.

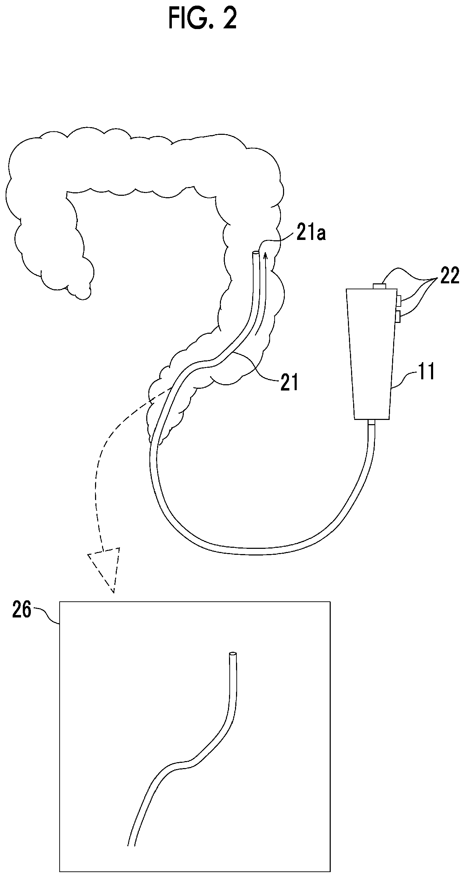

The CPU of the processor device 13 is a processor that causes the endoscope system of the present invention to function in cooperation with the memory or the like. The storage device stores control programs such as an operating system, various application programs, display data of various screens associated with these programs, and the like. The memory is a work memory for the CPU to execute processing. The CPU receives an input from an operating part 22 (see Fig. 2) of the endoscope 11 or the input device 18 and executes a program in the memory in order to operate various programs stored in the storage device. Accordingly, the functions of the respective parts (units) in the processor device 13 (such as the shape data collation unit 34 described below) are realized. In addition, an input method for the CPU may be based on voice input of a voice input unit (not shown) provided in the processor device 13.

The light source device 12 has a light source control processor (not shown) that controls a plurality of light emitting diodes (LEDs) that emit light in different wavelength ranges. The light source control processor independently controls the amounts of light of a violet LED (V-LED), a blue LED (B-LED), a green LED (G-LED), and a red LED (R-LED) that emit light corresponding to purple, blue, green, and red in a visible light region having a wavelength range of 380 to 780 nm, and causes the LEDs to emit illumination light having an emission spectrum adapted to each observation purpose. In addition, the LEDs correspond to light sources in the present specification.

The endoscope system 10 has a normal light mode and a special light mode as observation modes. In a case where an observation mode is the normal light mode, white light (first illumination light) is emitted as normal light, and a natural hue image is generated on the basis of an image signal obtained by radiating white light to image the part to be observed. Additionally, in a case where an observation mode is the special light mode, a special light (second illumination light) having a different emission spectrum from the white light is emitted, and an image in which the structure of a lesioned part, blood vessel, or mucous membrane is enhancement-processed is generated on the basis of an image signal obtained by radiating the special light to image the part to be observed. The processor device 13 displays on the display 17 various images generated according to each observation mode and information incidental to the images.

As shown in Fig. 2, the endoscope 11 has an insertion part 21 to be inserted into a body cavity of a subject (inside a lumen such as the large intestine) and the operating part 22 for operating the endoscope 11. The operating part 22 includes an angle knob that bends a distal end 21a of the insertion part 21 in upward-downward and leftward-rightward directions, an imaging button for imaging a part to be observed, a switch that executes the functions of the respective parts of the processor device 13, and the like. By operating the operating part 22, the distal end 21a of the insertion part 21 is directed in a desired direction, and an observation image obtained by imaging the inside of the body cavity of the subject is acquired by an imaging unit (not shown) having an imaging element such as a charge coupled device (CCD) built in the distal end 21a.

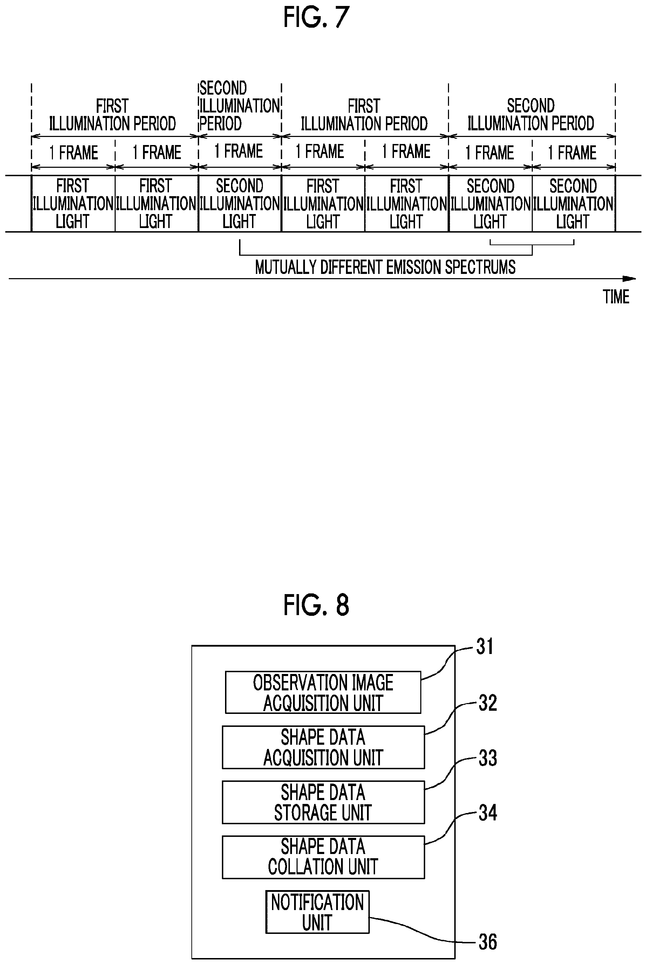

Additionally, a shape data acquisition unit 32 (see Fig. 8) detects a current value of an induced current generated in a coil (not shown) built in the insertion part 21 of the endoscope 11 due to the action of a magnetic field generated by the magnetic field generating device 14, and grasps the shape of the insertion part 21 of the endoscope 11 in the body cavity on the basis of the detected current value. Specifically, the known technique described in JP6633751B can be used.

In the present embodiment, by generating a magnetic field from the magnetic field generating device 14 in a short cycle, data on the shape of the insertion part 21 of the endoscope 11 is acquired in real time. On the basis of the data on the acquired shape, the shape data acquisition unit 32 acquires shape data representing the shape from the insertion position of the insertion part 21 of the endoscope 11 to the current position thereof with the movement of the distal end 21a of the insertion part 21 of the endoscope 11. In a case where the shape data is displayed on the display 17, it is preferable to generate and display the shape of the insertion part 21 currently being inserted as an image that can be visually discriminated as in a shape image 26 of Fig. 2. Hereinafter, in the present specification, the shape data and the shape image 26 are treated as the same, and in the drawings, the shape data is expressed as the shape image 26.

The light source device 12 has a light source control processor (not shown). The light source control processor has an automatic switching mode in which the white light and the special light are alternately switched in addition to the normal light mode and the special light mode. The automatic switching mode is a mode in which observation according to various purposes is performed by emitting illumination light in a predetermined wavelength range. For example, the automatic switching mode is a mode used for the purpose of detecting a plurality of lesions by alternately irradiating a part to be observed with the white light and the special light or generating an enhanced image for enhancing a lesioned part.

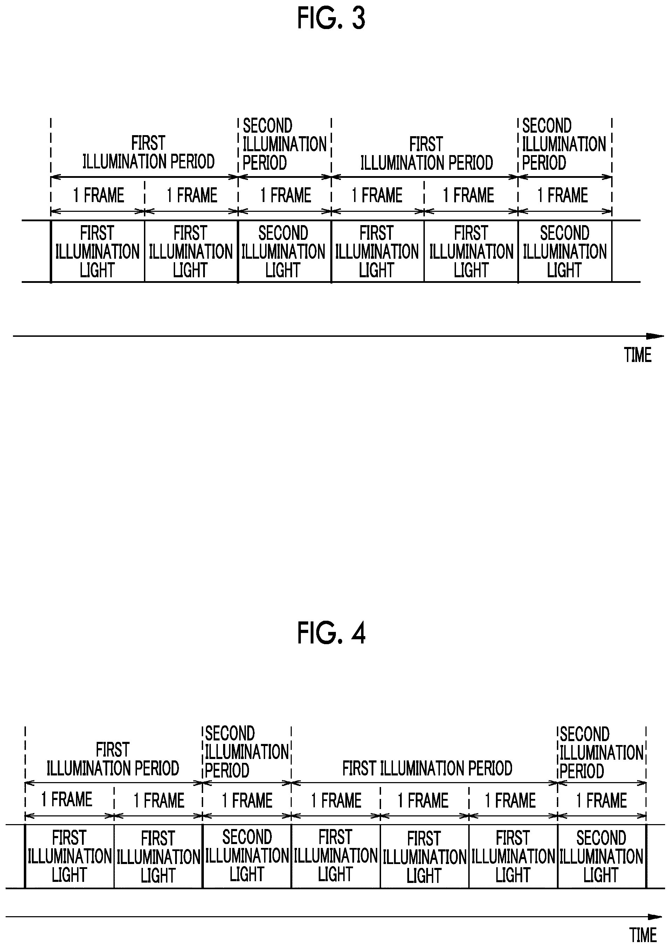

The light source control processor controls the light source in a predetermined light emission pattern. As shown in Fig. 3, in the automatic switching mode, the illumination light is controlled in a predetermined light emission pattern determined by each observation mode. The light source control processor automatically and alternately switches between a first illumination period and a second illumination period, causes the first illumination light to be emitted in a first light emission pattern in the first illumination period, and causes the second illumination light to be emitted in a second light emission pattern in the second illumination period. The first light emission pattern includes a first A light emission pattern and a first B light emission pattern. The second light emission pattern includes a second A light emission pattern, a second B light emission pattern, a second C light emission pattern, and a second D light emission pattern.

In a case where a predetermined illumination period is defined as one frame, as shown in Fig. 3, the first A light emission pattern is a light emission pattern in which the total number of frames obtained by summing the frames in each first illumination period is the same in all the first illumination periods. As shown in Fig. 4, the first B light emission pattern is a light emission pattern in which the total number of frames in one of the first illumination periods is different from the total number of frames in at least one other first illumination period.

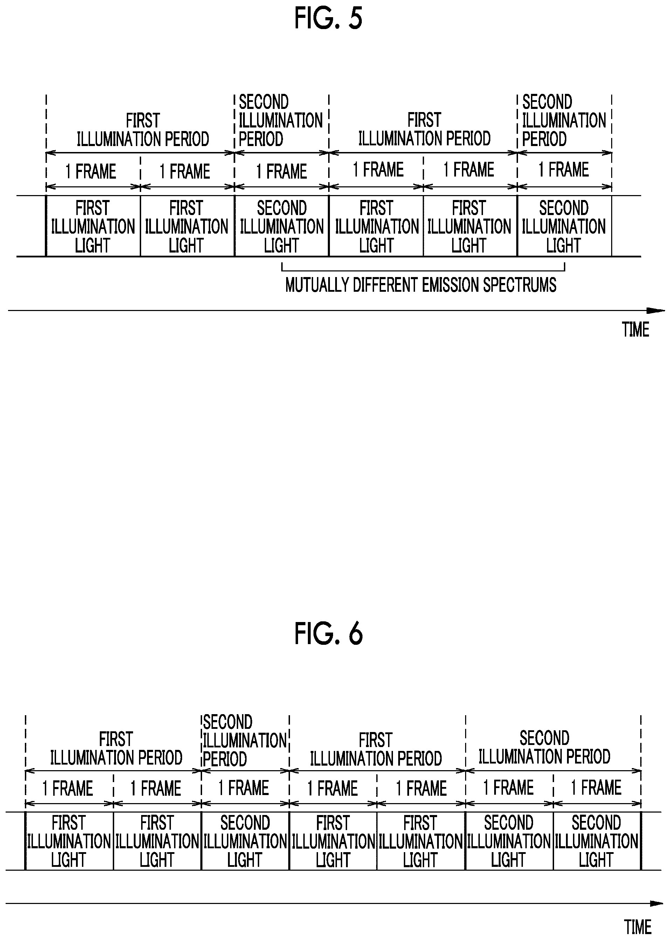

Additionally, as shown in Fig. 3, the second A light emission pattern is a light emission pattern in which the total number of frames in each second illumination period is the same in all the second illumination periods and the emission spectrum of the second illumination light in each second illumination period is the same in all the second illumination periods. As shown in Fig. 5, the second B light emission pattern is a light emission pattern in which the total number of frames in each second illumination period is the same in all the second illumination periods, and the emission spectrum of the second illumination light in one of the second illumination periods is different from the emission spectrum of the second illumination light in at least one other second illumination period. As shown in Fig. 6, the second C light emission pattern is a light emission pattern in which the total number of frames in one of the second illumination periods is different from the total number of frames in at least one other second illumination period and the emission spectrum of the second illumination light in each second illumination period is the same in all the second illumination periods. As shown in Fig. 7, the second D light emission pattern is a light emission pattern in which the total number of frames in one of the second illumination periods is different from the total number of frames in at least one other second illumination period and the emission spectrum of the second illumination light in one of the second illumination periods is different from the emission spectrum of the second illumination light in at least one other second illumination period.

In addition, the first illumination period is preferably longer than the second illumination period. For example, in Fig. 3, in a case where the first light emission pattern is a first A pattern and the second light emission pattern is a second A pattern, the first illumination period is two frames and the second illumination period is one frame.

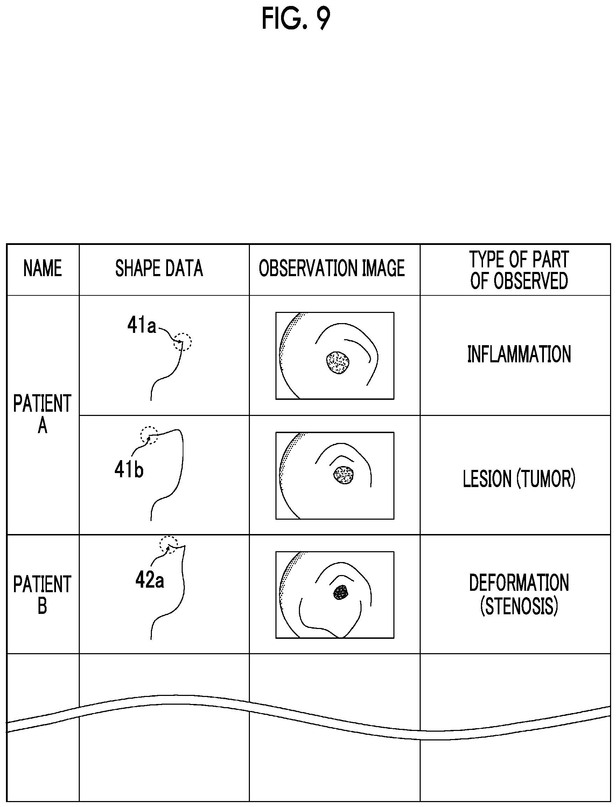

As shown in Fig. 8, the processor device 13 comprises an observation image acquisition unit 31, a shape data acquisition unit 32, a shape data storage unit 33, a shape data collation unit 34, and a notification unit 36. In a case where a user such as a doctor inserts the insertion part 21 of the endoscope 11 into the body cavity of the subject, the shape data acquisition unit 32 acquires the shape data of the insertion part 21 of the endoscope 11 in the body cavity with the movement of the distal end 21a of the insertion part 21. Additionally, the observation image acquisition unit 31 acquires an observation image of the part to be observed in a case where a release operation or the like for imaging the part to be observed is performed. The shape data storage unit 33 stores the shape data acquired the shape data acquisition unit 32 in a case where the user starts imaging the part to be observed or in a case where the user performs an operation of storing the shape data in order to observe any part to be observed again. Additionally, the shape data storage unit 33 stores the observation image of the part to be observed in association with the stored shape data. For example, as shown in Fig. 9, as the information stored in the shape data storage unit 33, for a patient A, regarding a part to be observed 41a, which is a first part to be observed in the insertion direction of the insertion part 21, "inflammation" is recorded as the shape data up to the position of the part to be observed 41a, the observation image of the part to be observed 41a, and the type of the part to be observed. Similarly, regarding a part to be observed 41b, which is a second part to be observed, the shape data up to the position of the part to be observed 41b, a "lesion (tumor)" is recorded as the observation image of the part to be observed 41b, and the type of the part to be observed. Additionally, for a patient B, regarding one part to be observed 42b, deformation (stenosis) is recorded as the shape data up to the position of the part to be observed 42b, the observation image of the part to be observed 42b, and the type of the part to be observed. In addition, the stenosis is a symptom that makes it difficult to insert the endoscope 11 due to the narrowing of the intestine, and such a part is also a target for observation. In addition, it is preferable that the shape data storage unit 33 store an image around a target part to be observed, an image of a landmark in the middle of a route, or the like in association with the shape data on the basis of not only the observation image of the part to be observed but also the image acquired with the insertion of the endoscope 11.

In Fig. 9, the type of the part to be observed that is stored in the shape data storage unit 33 reflects the result of the diagnosis of the part to be observed. The stored timing may be, for example, at the time of the user input after the part to be observed is diagnosed, or may be at the time of the input from a diagnosis support system (not shown) connected to the endoscope system 10. Alternatively, in a case where the part to be observed is analyzed on the basis of the observation image obtained by imaging the part to be observed using the special light, the input may be automatically performed according to the analysis result. In the diagnosis support system, diagnosis information such as the malignancy of the lesion or tumor included in the part to be observed may be stored as the diagnosis information observed in detail with respect to the part to be observed. The shape data storage unit 33 may store association information associated with the diagnosis information and the shape data.

The shape data collation unit 34 functions in a case where the part to be observed that has been observed in the past is observed again, such as the follow-up observation. For example, in a case where the shape data (first shape data) acquired in the past examination (first examination) is stored in the shape data storage unit 33, in the current examination (second examination) for performing the follow-up observation, the current shape data sequentially acquired by the shape data acquisition unit 32 and the first shape data are collated with each other in real time each time the shape data is acquired. In a case where the distal end 21a of the insertion part 21 eventually reaches the position of the part to be observed that has been observed in the past, the current shape data and the first shape data match each other. In a case where the current shape data and the first shape data match each other, the notification unit 36 determines that the distal end 21a of the insertion part 21 has reached the position of the target part to be observed, and notifies the user a result of determination. As a method of the notification, a notification sound may be output instead of displaying a notification icon 53 on the display 17. In addition, the match does not always mean a perfect match, and as long as a difference in a case where the shape data is compared is within a predetermined range, this case may be regarded as the match.

Hereinafter, the functions of the respective parts of the processor device 13 will be specifically described. In a case where the subject that is a target for endoscopy undergoes a first-time examination (first examination) for acquiring the shape data, the shape data is not stored in the shape data storage unit 33. In this case, the shape data is acquired in a case where the user observes the part to be observed along the progress of the examination. As shown in Fig. 9, each time the part to be observed is observed, the shape data storage unit 33 stores the shape data and the observation image of the part to be observed in order. In addition, the first shape data is the shape data acquired in the first examination. For example, the two shape data of the patient A in Fig. 9 are both the first shape data.

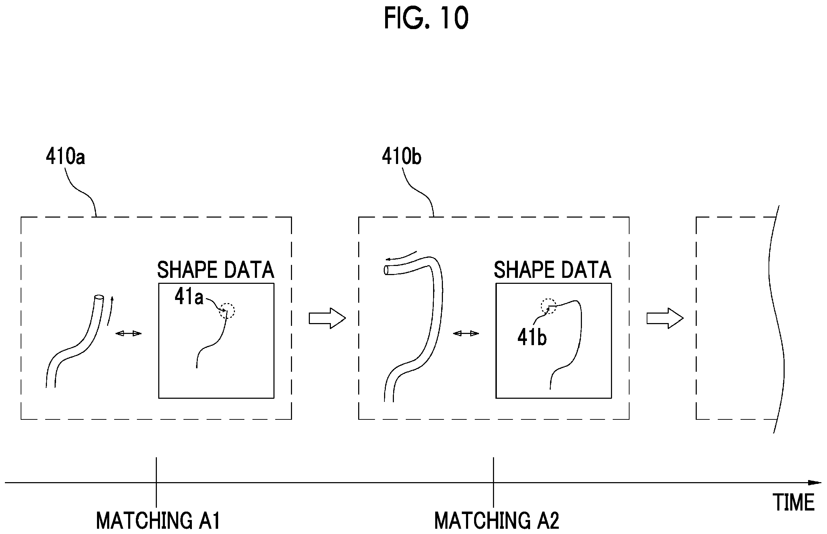

In a case where the follow-up observation (second examination) of the part to be observed that has been observed in the past examination, the shape data acquired with the movement of the distal end 21a of the insertion part 21 is collated with the first shape data stored in the shape data storage unit 33 in order from the first shape data on the part to be observed that is located on the near side with respect to the insertion direction of the insertion part 21. Fig. 10 is a diagram illustrating the functions of the shape data collation unit 34. In Fig. 10, time elapses from left to right. In the follow-up observation, in a case where the insertion part 21 of the endoscope 11 is inserted toward the part to be observed 41a, which is the first part to be observed of the patient A (see Fig. 9), the distal end 21a of the insertion part 21 reaches the vicinity of the position of the part to be observed 41a, and as in a comparison result 410a, the shape data of the insertion part 21 of the endoscope 11 in the current examination and the stored shape data up to the part to be observed 41a match each other (matching A1). In this case, the shape data collation unit 34 determines that the position of the part to be observed 41a that has been observed in the past has been reached. In a case where the observation of the part to be observed 41a is completed and the insertion part 21 of the endoscope 11 is inserted toward the part to be observed 41b, which is the second part to be observed, the distal end 21a of the insertion part 21 reaches the vicinity of the position of the part to be observed 41a, and as in a comparison result 410b, a current shape data and the stored shape data up to the part to be observed 41b match each other (matching A2), and the shape data collation unit 34 determine that the position of the part to be observed 41b that has been observed in the past has been reached. In addition, the shape data collation unit 34 collates the shape data acquired from time to time with the shape data on the target part to be observed even in a section not shown in Fig. 10 in real time.

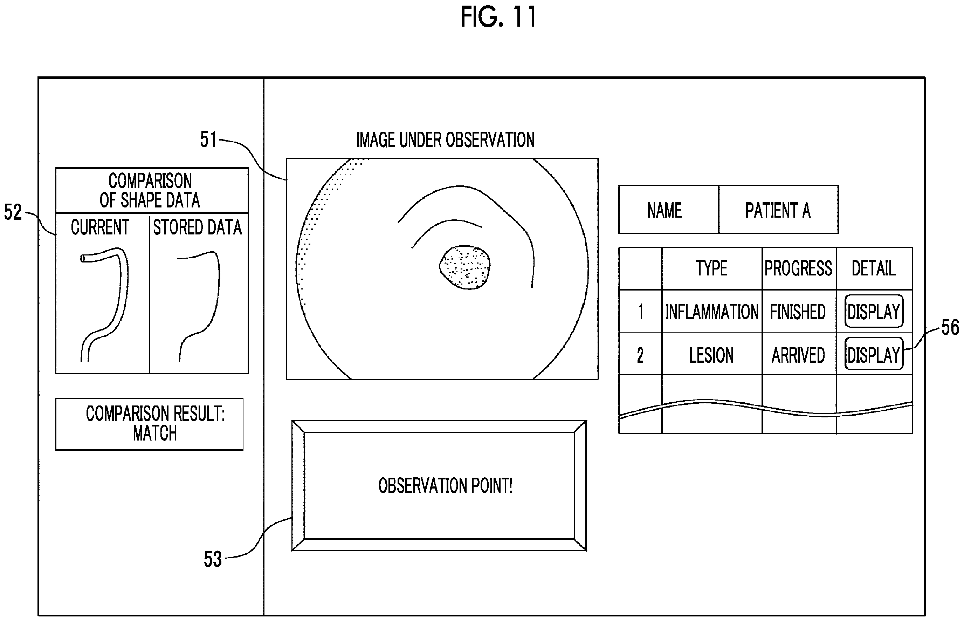

Fig. 11 is an example of the notification displayed on the display 17 by the notification unit 36. The observation image in the current examination is displayed as a motion picture 51 on the display 17. A comparison display region 52 displays the result obtained by comparing the current shape data with the stored shape data. In a case where the current shape data and the stored shape data match each other, the notification unit 36 displays on the screen the notification icon 53 informing the user a result of determination that the current position is near the target part to be observed.

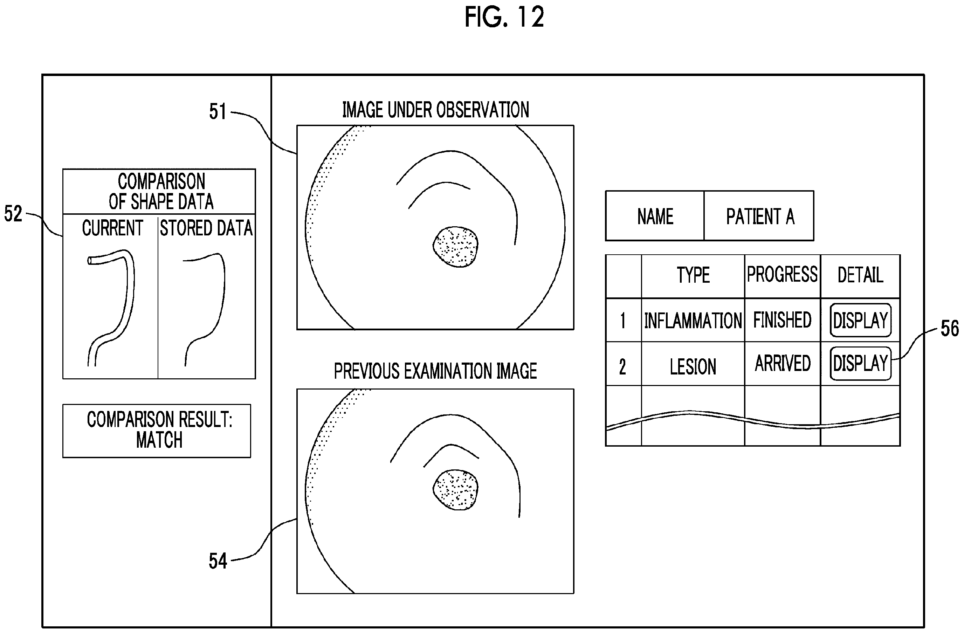

In addition, as a method of the notification, as shown in Fig. 12, since the previously observed image and the shape data are stored in association with each other in the shape data storage unit 33, the previously observed image (still image) 54 may be displayed in a case where the vicinity of the target part to be observed is reached. In this case, the previous part to be observed can be quickly specified by displaying an observation image 54 and displaying a plurality of images around the target part to be observed. In addition, the observation image and the observation image 54 displayed as the motion picture 51 are preferably white light images captured using the white light, but may be special light images captured using the special light.

In addition, in a case where diagnosis information corresponding to a specific shape data is stored in or is associated with the shape data storage unit 33, the operating part 22 of the endoscope 11 or the input device 18 can be used and called in a timely manner. For example, the diagnosis information on the part to be observed corresponding to an information display button 56 depressed by depressing the information display button 56 using the input device 18 is displayed on the display 17 shown in Fig. 11. Alternatively, in the second examination, the display may be automatically performed at the timing at which a detailed observation is performed.

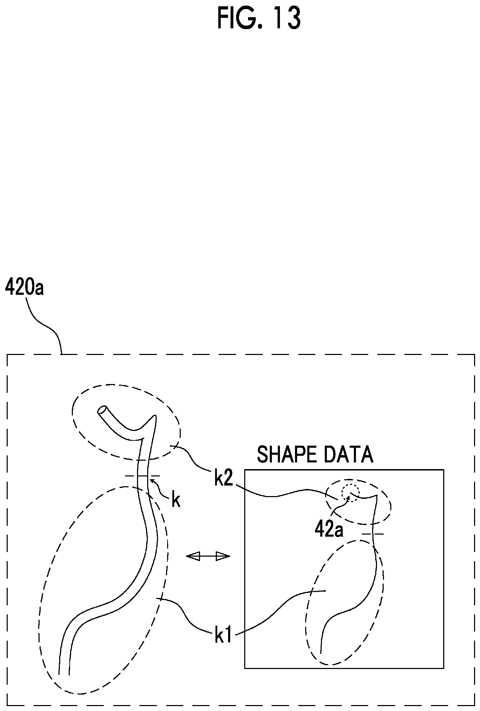

Fig. 13 is a diagram illustrating the functions of the shape data collation unit 34 in a case where the follow-up observation is performed on the patient B (see Fig. 9), but unlike Fig. 10, illustrating an example in which the current shape data and the stored first shape data do not match each other. Since there are parts that are likely to be deformed depending on organs to be observed, such as the large intestine, it is also considered that the lumen shape at the time of the previous examination and the lumen shape at the time of the follow-up observation are different from each other. As shown in Fig. 11, in the current examination, a range k1 up to the vicinity of a position k on the way to the target part to be observed and a range k2 after that in the shape data acquired in a case where the insertion part 21 of the endoscope 11 is inserted into a part to be observed 42a, which is the target part to be observed, are different from each other. In such a case, the shape data collation unit 34 determines that the current lumen shape is different from the previous lumen shape. Even in a case where the target part to be observed has not been reached, the notification unit 36 performs a notification in a case where it is determined that the current lumen shape is different from the previous lumen shape. As a method of the notification, as shown in Fig. 12, in addition to displaying the observation image, it is preferable to notify a user a result of determination that the current lumen shape is different from the previous lumen shape.

In Figs. 11 and 13, the shape data storage unit 33 preferably stores the shape data up to the position of the part to be observed that has been acquired in the second examination as a second shape data. The second shape data is used for a comparison target in the subsequent follow-up observations and the like.

In this way, in the endoscope system 10 in the present embodiment, in endoscopy targeting a part having poor visibility, it is easy to grasp the positions of parts where deformations such as inflamed spots and stenosis that are difficult to visually identify are occurring in addition to lesions and tumors. Moreover, even in a case where a current user in charge is different from the user in charge in the past examination, it is easy to grasp the position of the target part to be observed. Therefore, the endoscopy can be advanced without delay.

### Second Embodiment

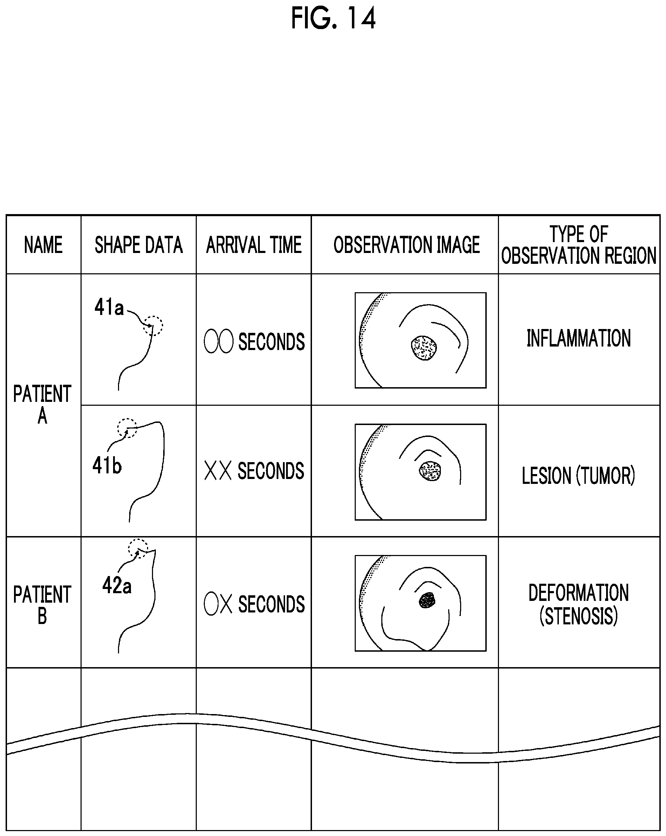

In the first embodiment, the current shape data and the first shape data stored in the shape data storage unit 33 are compared with each other to determine whether or not the target part to be observed has been reached. However, in the second embodiment, the shape data and the arrival time are used to perform the comparison. As shown in Fig. 14, in the first examination, the shape data storage unit 33 inserts the insertion part 21 into the body cavity in conformity with the shape data and the observation image, and then stores the arrival time that is the time at which the distal end 21a of the insertion part 21 reaches the position of the part to be observed. For example, for patient A, regarding he first part to be observed 41a, the shape data, the observation image of the part to be observed, and the type of the part to be observed are the same as those in the first embodiment. However, in addition to these, the arrival time that is the time at which the distal end 21a reaches the position of the part to be observed 41a is recorded as "∘∘ seconds".

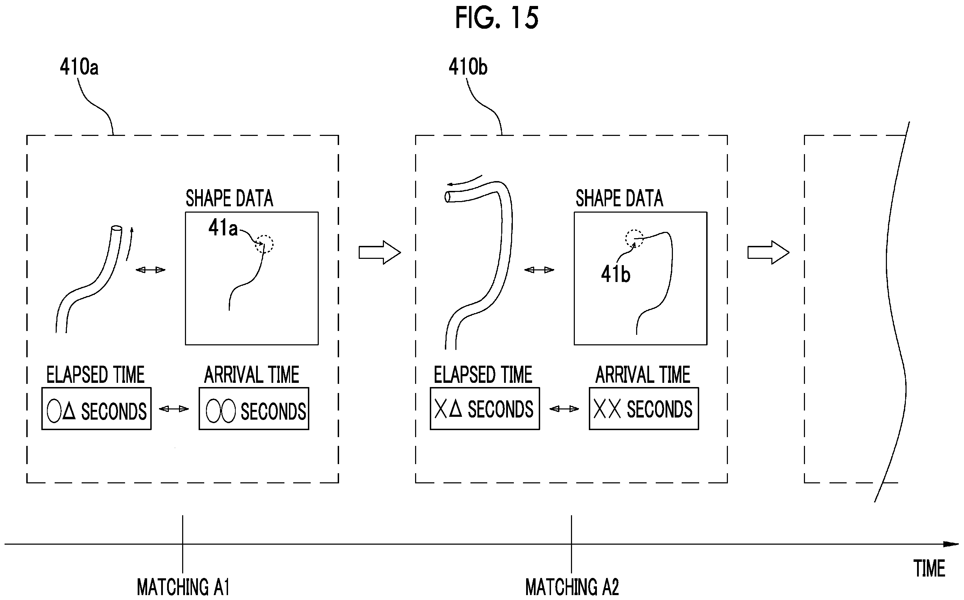

In a case where the follow-up observation (second examination) is performed, similarly to the first embodiment, the shape data acquired with the movement of the insertion part 21 of the endoscope 11 and the elapsed time since the insertion part 21 are inserted into the body cavity are compared with the shape data and the arrival time to the target part to be observed that has been stored in the shape data storage unit 33. As shown in Fig. 15, in a case where the current shape data and the elapsed time match the stored shape data and arrival time, the shape data collation unit 34 determines that the vicinity of the target part to be observed has been reached. In this case, the notification unit 36 notifies the user a result of determination that the vicinity of the target part to be observed has been reached. In addition, regarding the time (arrival time, elapsed time), the insertion time is not always constant. Therefore, in a case where the difference between the elapsed time and the arrival time is within any time range, it is determined that the arrival times match each other. Additionally, for the purpose of searching for the past part to be observed, it is preferable to perform a notification before the position of the target part to be observed is reached. Therefore, in a case where the current shape data and the previous shape data match each other, and in a case where the time obtained by subtracting any time from the arrival time is equal to the elapsed time, it may be determined that the vicinity of the target part to be observed has been reached.

### Third Embodiment

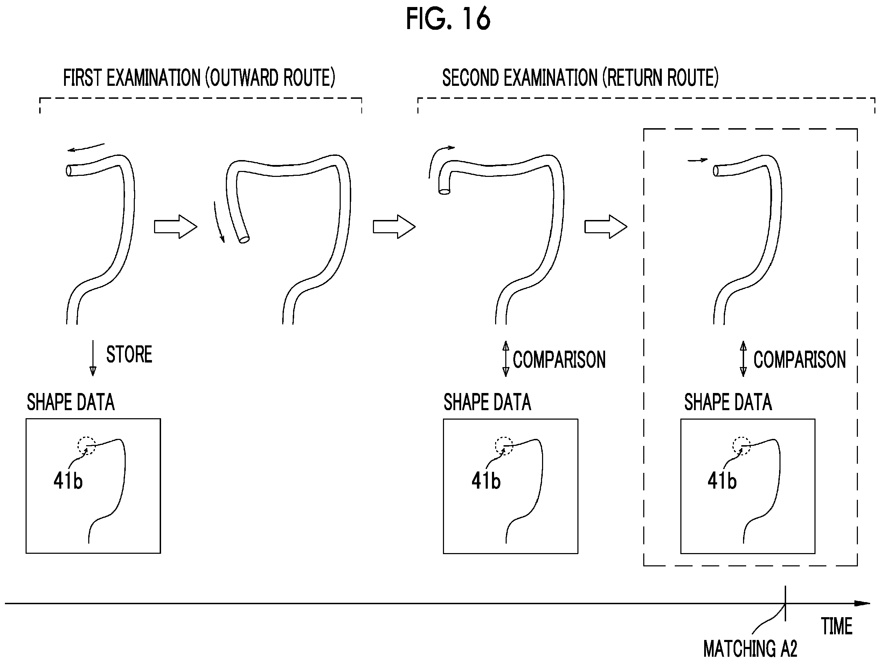

In the first and second embodiments, a scene of the follow-up observations is assumed as the second examination. However, in the third embodiment, an examination scene where in-path screening is performed in an outward route in the insertion direction of the endoscope 11 and then a detailed observation is performed on a return route is assumed. Fig. 16 shows an example in which the position of the part to be observed 41b is searched for the patient A in the present embodiment. In the outward route (first examination), the shape data storage unit 33 stores the shape data up to the position of the part to be observed 41b as the first shape data. In a case where the innermost part of the examination range is reached in the examination of the outward route, the shape data collation unit 34 collates the shape data acquired in real time in the return route (second examination) with the first shape data until the shape data matches the first shape data. In a case where the shape data acquired in the return route matches the first shape data (matching A2), the notification unit 36 notifies the user a result of determination that the vicinity of the target part to be observed has been reached.

It goes without saying that the present invention is not limited to the above respective embodiments and modification examples, and can have various configurations as long as the invention does not depart from the scope of the present invention. For example, it is also possible to appropriately combine the above embodiments and modification examples with each other. Moreover, the present invention extends to a storage medium for storing a program in addition to the programs.

In the present embodiment, the hardware structures of the processing unit, which executes various processing, such as the observation image acquisition unit 31 and the shape data collation unit 34 are various processors as shown next. The various processors include a central processing unit (CPU) that is a general-purpose processor that executes software (programs) to function as various processing units, a programmable logic device (PLD), which is a processor capable of changing the circuit configuration after manufacturing, such as a field programmable gate array (FPGA), a dedicated electric circuit that is a processor having a circuit configuration designed in a dedicated manner to execute various processing, and the like.

One processing unit may be constituted of one of the various processors, or may be constituted of a combination (for example, a plurality of FPGAs or a combination of a CPU and an FPGA) of two or more processors of the same type or different types. Additionally, a plurality of processing units may be constituted of one processor. As an example in which the plurality of processing units is constituted of one processor, firstly, as represented by a computer such as a client or a server, there is a form in which one processor is configured by a combination of one or more CPUs and software and this processor functions as the plurality of processing units. Secondly, as represented by system on chip (SoC), there is a form in which a processor that realizes the functions of the entire system including a plurality of processing units with one integrated circuit (IC) chip is used. In this way, the various processing units are configured using one or more of the various processors as the hardware structure.

Moreover, the hardware structure of the various processors is, more specifically, an electric circuit (circuitry) in a form in which circuit elements such as semiconductor elements are combined together.

### Explanation of References

10: endoscope system
11: endoscope
12: light source device
13: processor device
14: magnetic field generating device
17: display
18: input device
21: insertion part
21a: distal end
22: operating part
26: shape image
31: observation image acquisition unit
32: shape data acquisition unit
33: shape data storage unit
34: shape data collation unit
36: notification unit
41a: part to be observed
41b: part to be observed
42a: part to be observed
51: motion picture
52: comparison display region
53: notification icon
54: observation image
56: information display button
410a: comparison result
410b: comparison result
420a: comparison result

## Claims

1. An endoscope system comprising:
a processor (13) configured to acquire shape data of an insertion part (21) of an endoscope (11) in a body cavity in an examination using the endoscope with movement of a distal end (21a) of the insertion part; and
a memory (33) configured to store the shape data,
wherein the processor is configured to store the shape data at a position of an object to be observed in a first examination as first shape data in the memory, and
to determine that the distal end of the insertion part has reached the position of the object to be observed and provides notification of a result of determination in a case where the shape data acquired in a second examination after the first examination matches the first shape data; and wherein the processor is further configured to store in the memory an arrival time, which is a time at which the distal end of the insertion part reaches the position of the object to be observed after the insertion part is inserted into the body cavity, and to perform a notification on the basis of the arrival time

2. The endoscope system according to claim 1, further comprising:
a display (17),
wherein the processor is configured to store an observation image obtained by imaging the object to be observed and the first shape data in association with each other in the memory, and
to display the observation image on the display in a case where the shape data acquired in the second examination matches the first shape data.

3. The endoscope system according to claim 1 or 2,
wherein the processor is configured to compare the shape data with the first shape data each time the shape data is acquired, in the second examination.

4. The endoscope system according to any one of claims 1 to 3,
wherein the processor is configured to store the shape data, which has matched the first shape data, in the second examination as second shape data in the memory.

5. The endoscope system according to any one of claims 1 to 4,
wherein the processor is configured to display diagnosis information of the object to be observed, which is associated with the first shape data, on the display.

6. The endoscope system according to any one of claims 1 to 5, further comprising:
a light source control processor,
wherein the light source control processor is configured to control a light source that emits first illumination light and second illumination light having a different emission spectrum from the first illumination light.

7. The endoscope system according to claim 6,
wherein the first illumination light is white light, and
the observation image is an image captured by irradiating the object to be observed with the first illumination light.

8. The endoscope system according to claim 6 or 7,
wherein the light source control processor
causes the first illumination light to be emitted in a first illumination period,
causes the second illumination light to be emitted in a second illumination period, and
in a case where the first illumination period and the second illumination period are automatically and alternately switched and in a case where a predetermined illumination period is defined as one frame, controls the first illumination light in a first light emission pattern having a first A light emission pattern in which a total number of frames obtained by summing the frames in each first illumination period is the same in all the first illumination periods, and a first B light emission pattern in which the total number of frames in one of the first illumination periods is different from the total number of frames in at least one other first illumination period.

9. The endoscope system according to claim 8,
wherein the light source control processor is configured to control the second illumination light in a second light emission pattern,
the second light emission pattern has a second A light emission pattern, a second Blight emission pattern, a second C light emission pattern, and a second D light emission pattern,
the second A light emission pattern is a light emission pattern in which a total number of frames in each second illumination period is the same in all the second illumination periods and an emission spectrum of the second illumination light in each second illumination period is the same in all the second illumination periods,
the second B light emission pattern is a light emission pattern in which the total number of frames in each second illumination period is the same in all the second illumination periods, and the emission spectrum of the second illumination light in one of the second illumination periods is different from the emission spectrum of the second illumination light in at least one other second illumination period,
the second C light emission pattern is a light emission pattern in which the total number of frames in one of the second illumination periods is different from the total number of frames in at least one other second illumination period and the emission spectrum of the second illumination light in each second illumination period is the same in all the second illumination periods, and
the second D light emission pattern is a light emission pattern in which the total number of frames in one of the second illumination periods is different from the total number of frames in at least one other second illumination period and the emission spectrum of the second illumination light in one of the second illumination periods is different from the emission spectrum of the second illumination light in at least one other second illumination period.

## Patentansprüche

1. Endoskopsystem, das Folgendes umfasst:
einen Prozessor (13), der dazu konfiguriert ist, Formdaten eines Einsteckteils (21) eines Endoskops (11) in einer Körperhöhle bei einer Untersuchung unter Verwendung des Endoskops mit Bewegung eines distalen Endes (21a) des Einsteckteils zu erfassen; und einen
Speicher (33), der dazu konfiguriert ist, die Formdaten zu speichern,
wobei der Prozessor dazu konfiguriert ist, die Formdaten an einer Position eines bei einer ersten Untersuchung zu beobachtenden Objekts als erste Formdaten in dem Speicher zu speichern und
zu bestimmen, dass das distale Ende des Einsteckteils die Position des zu beobachtenden Objekts erreicht hat, und eine Benachrichtigung über ein Bestimmungsergebnis in einem Fall bereitzustellen, in dem die bei einer zweiten Untersuchung nach der ersten Untersuchung erfassten Formdaten mit den ersten Formdaten übereinstimmen; und wobei der Prozessor ferner dazu konfiguriert ist, eine Ankunftszeit, die ein Zeitpunkt ist, zu dem das distale Ende des Einsteckteils die Position des zu beobachtenden Objekts erreicht, nachdem das Einsteckteil in die Körperhöhle eingeführt wurde, im Speicher zu speichern und auf der Grundlage der Ankunftszeit eine Benachrichtigung durchzuführen

2. Endoskopsystem nach Anspruch 1, ferner umfassend:
eine Anzeige (17),
wobei der Prozessor dazu konfiguriert ist, ein Beobachtungsbild, das durch Abbilden des zu beobachtenden Objekts erhalten wird, und die ersten Formdaten in Verbindung miteinander in dem Speicher zu speichern, und
das Beobachtungsbild auf der Anzeige in einem Fall anzuzeigen, in dem die in der zweiten Untersuchung erfassten Formdaten mit den ersten Formdaten übereinstimmen.

3. Endoskopsystem nach Anspruch 1 oder 2,
wobei der Prozessor dazu konfiguriert ist, die Formdaten bei jeder Erfassung der Formdaten bei der zweiten Untersuchung mit den ersten Formdaten zu vergleichen.

4. Endoskopsystem nach einem der Ansprüche 1 bis 3,
wobei der Prozessor dazu konfiguriert ist, die Formdaten, die mit den ersten Formdaten übereinstimmen, bei der zweiten Untersuchung als zweite Formdaten im Speicher zu speichern.

5. Endoskopsystem nach einem der Ansprüche 1 bis 4,
wobei der Prozessor dazu konfiguriert ist, Diagnoseinformationen des zu beobachtenden Objekts, die mit den ersten Formdaten verknüpft sind, auf dem Display anzuzeigen.

6. Endoskopsystem nach einem der Ansprüche 1 bis 5, ferner umfassend:
einen Lichtquellen-Steuerungsprozessor,
wobei der Lichtquellen-Steuerungsprozessor dazu konfiguriert ist, eine Lichtquelle zu steuern, die ein erstes Beleuchtungslicht und ein zweites Beleuchtungslicht mit einem vom ersten Beleuchtungslicht verschiedenen Emissionsspektrum emittiert.

7. Endoskopsystem nach Anspruch 6,
wobei das erste Beleuchtungslicht weißes Licht ist und
das Beobachtungsbild ein Bild ist, das durch Bestrahlen des zu beobachtenden Objekts mit dem ersten Beleuchtungslicht aufgenommen wird.

8. Endoskopsystem nach Anspruch 6 oder 7,
wobei der Lichtquellen-Steuerungsprozessor
veranlasst, dass das erste Beleuchtungslicht in einer ersten Beleuchtungsperiode emittiert wird,
veranlasst, dass das zweite Beleuchtungslicht in einer zweiten Beleuchtungsperiode emittiert wird, und
in einem Fall, in dem die erste Beleuchtungsperiode und die zweite Beleuchtungsperiode automatisch und abwechselnd umgeschaltet werden, und in einem Fall, in dem eine vorbestimmte Beleuchtungsperiode als ein Frame definiert ist, das erste Beleuchtungslicht in einem ersten Lichtemissionsmuster mit einem ersten A-Lichtemissionsmuster steuert, bei dem eine Gesamtzahl von Frames, die durch Summieren der Frames in jeder ersten Beleuchtungsperiode erhalten wird, in allen ersten Beleuchtungsperioden gleich ist, und mit einem ersten B-Lichtemissionsmuster steuert, bei dem die Gesamtzahl der Frames in einer der ersten Beleuchtungsperioden von der Gesamtzahl der Frames in zumindest einer anderen ersten Beleuchtungsperiode verschieden ist.

9. Endoskopsystem nach Anspruch 8,
wobei der Lichtquellen-Steuerungsprozessor dazu konfiguriert ist, das zweite Beleuchtungslicht in einem zweiten Lichtemissionsmuster zu steuern,
wobei das zweite Lichtemissionsmuster ein zweites A-Lichtemissionsmuster, ein zweites B-Lichtemissionsmuster, ein zweites C-Lichtemissionsmuster und ein zweites D-Lichtemissionsmuster aufweist,
wobei das zweite A-Lichtemissionsmuster ein Lichtemissionsmuster ist, bei dem eine Gesamtzahl von Frames in jeder zweiten Beleuchtungsperiode in allen zweiten Beleuchtungsperioden gleich ist und ein Emissionsspektrum des zweiten Beleuchtungslichts in jeder zweiten Beleuchtungsperiode in allen zweiten Beleuchtungsperioden gleich ist,
wobei das zweite B-Lichtemissionsmuster ein Lichtemissionsmuster ist, bei dem die Gesamtzahl der Frames in jeder zweiten Beleuchtungsperiode in allen zweiten Beleuchtungsperioden gleich ist und das Emissionsspektrum des zweiten Beleuchtungslichts in einer der zweiten Beleuchtungsperioden sich vom Emissionsspektrum des zweiten Beleuchtungslichts in zumindest einer anderen zweiten Beleuchtungsperiode unterscheidet,
wobei das zweite C-Lichtemissionsmuster ein Lichtemissionsmuster ist, bei dem die Gesamtzahl der Frames in einer der zweiten Beleuchtungsperioden von der Gesamtzahl der Frames in zumindest einer anderen zweiten Beleuchtungsperiode verschieden ist und das Emissionsspektrum des zweiten Beleuchtungslichts in jeder zweiten Beleuchtungsperiode in allen zweiten Beleuchtungsperioden gleich ist, und
wobei das zweite D-Lichtemissionsmuster ein Lichtemissionsmuster ist, bei dem die Gesamtzahl der Frames in einer der zweiten Beleuchtungsperioden von der Gesamtzahl der Frames in zumindest einer anderen zweiten Beleuchtungsperiode verschieden ist und das Emissionsspektrum des zweiten Beleuchtungslichts in einer der zweiten Beleuchtungsperioden von dem Emissionsspektrum des zweiten Beleuchtungslichts in zumindest einer anderen zweiten Beleuchtungsperiode verschieden ist.

## Revendications

1. Un système d'endoscope comprenant :
un processeur (13) configuré pour acquérir des données de forme d'une pièce d'insertion (21) d'un endoscope (11) dans une cavité corporelle lors d'un examen utilisant l'endoscope avec un mouvement d'une extrémité distale (21a) de la pièce d'insertion ; et.
une mémoire (33) configurée pour stocker les données de forme,
dans lequel le processeur est configuré pour stocker les données de forme à une position d'un objet à observer dans un premier examen en tant que premières données de forme dans la mémoire, et
pour déterminer que l'extrémité distale de la pièce d'insertion a atteint la position de l'objet à observer et fournit une notification du résultat de la détermination dans le cas où les données de forme acquises lors d'un deuxième examen après le premier examen correspondent aux premières données de forme ; et dans lequel le processeur est en outre configuré pour stocker dans la mémoire un temps d'arrivée, qui est un temps auquel l'extrémité distale de la pièce d'insertion atteint la position de l'objet à observer après que la pièce d'insertion est insérée dans la cavité corporelle, et pour effectuer une notification sur la base du temps d'arrivée.

2. Le système d'endoscope selon la revendication 1, comprenant en outre :
un afficheur (17),
dans lequel le processeur est configuré pour stocker une image d'observation obtenue en imageant l'objet à observer et les premières données de forme en association l'une avec l'autre dans la mémoire, et
pour afficher l'image d'observation sur l'afficheur dans un cas où les données de forme acquises lors du deuxième examen correspondent aux premières données de forme.

3. Le système d'endoscope selon la revendication 1 ou 2,
dans lequel le processeur est configuré pour comparer les données de forme avec les premières données de forme à chaque fois que les données de forme sont acquises, lors du deuxième examen.

4. Le système d'endoscope selon l'une quelconque des revendications 1 à 3,
dans lequel le processeur est configuré pour stocker les données de forme, qui ont correspondu aux premières données de forme, dans le deuxième examen, en tant que deuxièmes données de forme dans la mémoire.

5. Le système d'endoscope selon l'une quelconque des revendications 1 à 4,
dans lequel le processeur est configuré pour afficher sur l'afficheur des informations de diagnose de l'objet à observer, qui sont associées aux premières données de forme.

6. Le système d'endoscope selon l'une quelconque des revendications 1 à 5, comprenant en outre :
un processeur de contrôle de source lumineuse,
dans lequel le processeur de contrôle de source lumineuse est configuré pour contrôler une source lumineuse qui émet une première lumière d'illumination et une deuxième lumière d'illumination ayant un spectre d'émission différent de celui de la première lumière d'illumination.

7. Le système d'endoscope selon la revendication 6,
dans lequel la première lumière d'illumination est une lumière blanche, et
l'image d'observation est une image capturée en irradiant l'objet à observer avec la première lumière d'illumination.

8. Le système d'endoscope selon la revendication 6 ou 7,
dans lequel le processeur de contrôle de source lumineuse
cause la première lumière d'illumination à être émise dans une première période d'illumination,
cause l'émission de la deuxième lumière d'illumination dans une deuxième période d'illumination, et
dans le cas où la première période d'illumination et la deuxième période d'illumination sont commutées automatiquement et alternativement et dans le cas où une période d'illumination prédéterminée est définie comme une trame, contrôle la première lumière d'illumination dans un premier schéma d'émission de lumière ayant un premier schéma A d'émission de lumière dans lequel un nombre total de trames obtenu en additionnant les trames dans chaque première période d'illumination est le même dans toutes les premières périodes d'illumination, et un premier schéma B d'émission de lumière dans lequel le nombre total de trames dans l'une des premières périodes d'illumination est différent du nombre total de trames dans au moins une autre première période d'illumination.

9. Le système d'endoscope selon la revendication 8,
dans lequel le processeur de contrôle de source de lumière est configuré pour contrôler la deuxième lumière d'illumination dans un deuxième schéma d'émission de lumière,
le deuxième schéma d'émission de lumière comporte un deuxième schéma A d'émission de lumière, un deuxième schéma B d'émission de lumière, un deuxième schéma C d'émission de lumière, et un deuxième schéma D d'émission de lumière,
le deuxième schéma A d'émission de lumière étant un schéma d'émission de lumière dans lequel le nombre total de trames dans chaque deuxième période d'illumination est le même dans toutes les deuxième périodes d'illumination et un spectre d'émission de la deuxième lumière d'illumination dans chaque deuxième période d'illumination est le même dans toutes les deuxième périodes d'illumination,
le deuxième schéma B d'émission de lumière étant un schéma d'émission de lumière dans lequel le nombre total de trames dans chaque deuxième période d'illumination est le même dans toutes les deuxièmes périodes d'illumination, et le spectre d'émission de la deuxième lumière d'illumination dans l'une des deuxièmes périodes d'illumination est différent du spectre d'émission de la deuxième lumière d'illumination dans au moins une autre deuxième période d'illumination,
le deuxième schéma C d'émission de lumière étant un schéma d'émission de lumière dans lequel le nombre total de trames dans l'une des deuxièmes périodes d'illumination est différent du nombre total de trames dans au moins une autre deuxième période d'illumination et le spectre d'émission de la deuxième lumière d'illumination dans chaque deuxième période d'illumination est le même dans toutes les deuxièmes périodes d'illumination, et
le deuxième schéma D d'émission de lumière étant un schéma d'émission de lumière dans lequel le nombre total de trames dans l'une des deuxièmes périodes d'illumination est différent du nombre total de trames dans au moins une autre deuxième période d'illumination et le spectre d'émission de la deuxième lumière d'illumination dans l'une des deuxièmes périodes d'illumination est différent du spectre d'émission de la deuxième lumière d'illumination dans au moins une autre deuxième période d'illumination.
